# EUROPÄISCHE PATENTSCHRIFT

(11) **EP 1 924 542 B1**
(45) Veröffentlichungstag und Bekanntmachung des Hinweises auf die Patenterteilung: **27.04.2011**
(21) Anmeldenummer: 06793142.8
(22) Anmeldetag: 01.09.2006
(51) Int. Cl.: C07C 29/70, C22B 26/22, C01F 5/30, C01F 7/48, C07F 3/02

(54) **ERZEUGUNG VON AKTIVIERTEM ERDALKALIMETALL, INSBESONDERE MAGNESIUM, ZUR HERSTELLUNG VON ORGANO-ERDALKALIMETALL-VERBINDUNGEN**
PREPARATION OF ACTIVATED ALKALINE EARTH METAL, IN PARTICULAR MAGNESIUM, FOR USE IN THE PRODUCTION OF ORGANO-ALKALINE- EARTH-METAL COMPOUNDS
PRÉPARATION DE MÉTAL ALCALINO-TERREUX ACTIVÉ, NOTAMMENT MAGNESIUM, UTILISÉ POUR PRODUIRE DES COMPOSÉS DE MÉTAL ORGANO-ALCALINO-TERREUX

(30) Priorität: 01.09.2005 DE 102005041784
(43) Veröffentlichungstag der Anmeldung: 28.05.2008
(73) Patentinhaber: Chemetall GmbH, 60487 Frankfurt am Main (DE)
(72) Erfinder: DIETZ, Rainer, 63329 Egelsbach (DE); EMMEL, Ute, 65929 Frankfurt am Main (DE); WIETELMANN, Ulrich, 61381 Friedrichsdorf (DE); LISCHKA, Uwe, 60437 Frankfurt am Main (DE)
(74) Vertreter: Uppena, Franz
(86) Internationale Anmeldenummer: PCT/EP2006/065917
(87) Internationale Veröffentlichungsnummer: WO 2007/026016

(56) Entgegenhaltungen:
- WO-A-91/05608
- US-A- 3 758 620
- US-A- 5 093 443
- DATABASE WPI Week 198602 Derwent Publications Ltd., London, GB; AN 1986-012166 XP002139673 & SU 477 626 A (VAVILOV V V) 15. August 1985 (1985-08-15)

## Beschreibung

Gegenstand der vorliegenden Erfindung ist ein aktiviertes Erdalkalimetall, ein Verfahren zu dessen Herstellung und die Anwendung des aktivierten Erdalkalimetalls.

Erdalkalimetall, insbesondere Magnesium, ist in verschiedensten Lieferformen, wie beispielsweise Pulvern, Spänen, Granulaten, Barren etc., auch im industriellen Maßstab, verfügbar. Magnesium dient in der Chemie beispielsweise als Rohstoff für die Herstellung von Dialkylmagnesiumverbindungen, Alkylmagnesiumhalogeniden, insbesondere Grignard-Verbindungen, Magnesiumalkoxiden, Magnesiumbis-(dialkyl)amiden und Magnesiumhydrid.

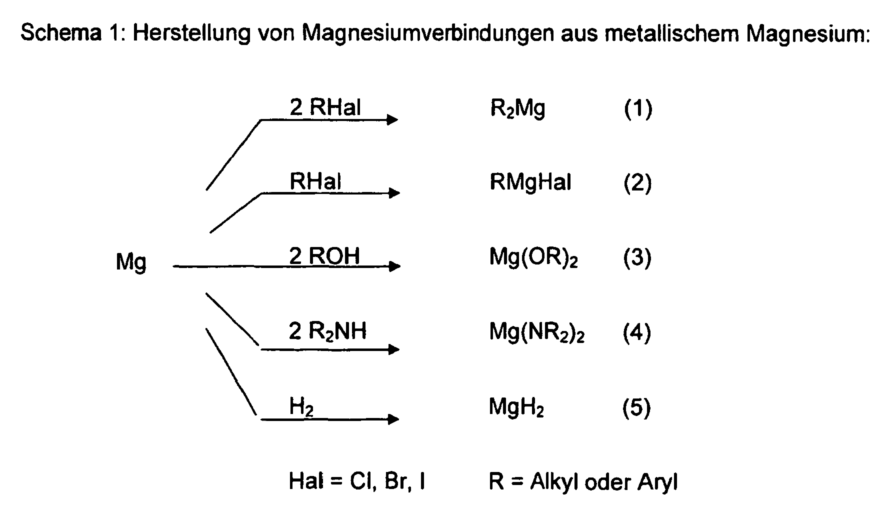

Die Umsetzungen gemäß (1) bis (4) werden normalerweise in einem aprotischen Lösemittel oder Lösemittelgemisch durchgeführt. Bei der praktischen Durchführung der genannten Reaktionen muss in der Regel festgestellt werden, dass die gewünschte Umsetzung gehemmt ist, das heißt, es findet keine Reaktion statt. Das kommerziell verfügbare metallische Magnesium ist nämlich mit einer kompakten, für die oben in Schema 1 aufgeführten Reagenzien undurchdringlichen Schicht an Korrosionsprodukten überzogen. Um diese Schicht zu entfernen, wurden für den Fall der Grignardsynthese (Reaktion (2)) in koordinierenden Lösemitteln, beispielsweise Tetrahydrofuran (THF), verschiedene Aktivierungsmethoden entwickelt (Überblick: U. Tilstam u. H. Weinmann, Org. Proc. Dev. 2002, 6, 909-910):
1. Auflösen der Oxidschicht in Mineralsäuren.
   Nachteil: Dieses Verfahren benötigt nachfolgend einen Waschschritt unter Inertgas, bei dem alle Säurespuren entfernt werden. Dies ist im industriellen Maßstab nur sehr schwierig zu realisieren.
2. Trockenes Rühren oder Mahlen: dadurch wird die Schutzschicht stellenweise mechanisch entfernt.
   Nachteil: Auch dieses Verfahren ist im größeren Maßstab kaum durchführbar, da das oft aus Glas oder Glasemaille bestehende Behältermaterial dadurch geschädigt wird und Rührorgane in der Regel nicht tief genug in einen Rührkessel hereinreichen.
3. Addieren von fertigem Grignardreagenz. diese Methode ist für mehrere aufeinander folgende Ansätze ein probates Mittel.
   Nachteil: In der Regel steht am Anfang einer Kampagne aber kein Fertigprodukt zur Verfügung.
4. Aktivierung durch lod oder Dibromethan.
   Nachteil: lod ist stark ätzend und Dibromethan ein Karzinogen. Beide Mittel sind deshalb für die industrielle Synthese wenig geeignet.
5. Aluminiumhydridaktivierung: der Zusatz von Natrium-bis(2-methoxyethoxy) aluminiumhydrid oder Diisobutylaluminiumhydrid (DiBAH) soll Magnesium in etherischen Lösemitteln wie Diethylether oder THF für die nachfolgende Reaktion mit organischen Halogeniden aktivieren.
   Nachteil: In Diethylether sind dazu 5 bis 12 mol% Aluminiumverbindung (bezogen auf eingesetztes Magnesiummetall) notwendig (US-A-3,758,620); in THF wird 1 mol% benötigt.

Viele der in Schema 1 aufgeführten Verbindungen, insbesondere die Dialkylmagnesiumverbindungen (1) und Magnesiumalkoholate (3), werden vor allem für Anwendungen in der Olefin- oder Dienpolymerisation benötigt. Für diese Verwendung dürfen die eingesetzten Magnesiumverbindungen keinerlei Donorlösemittel, insbesondere keine Ether enthalten. Vielmehr werden für einen solchen Zweck entweder trockene, lösemittelfreie Produkte oder bevorzugt Lösungen in nicht-koordinierenden Lösemittelsystemen, insbesondere in aromatischen oder aliphatischen Kohlenwasserstoffen, eingesetzt. Ferner sollten die für die Polymerisationsinitiation eingesetzten Verbindungen, beispielsweise Ziegler-Natta-Katalysatoren, keine Verunreinigungen aufweisen, die sich nachteilig auf die Anwendungseigenschaften auswirken. Eine solcherweise nachteilige Verunreinigung ist beispielsweise lod (EP-A-997 451).

Aufgabe der Erfindung ist es daher, ein Verfahren zur Herstellung des aktivierten Erdalkalimetalls bereitzustellen, das die Nachteile des Standes der Technik überwindet.

Erfindungsgemäß wird die Aufgabe überraschenderweise durch die Merkmale des Hauptanspruchs gelöst. Vorzugsweise Ausgestaltungen finden sich in den Unteransprüchen.

Aktiviertes Erdalkalimetall im Sinne der Erfindung bedeutet, dass das Erdalkalimetall mit organischen Halogeniden und/oder Alkoholen unverzögert reagiert, das heißt mindestens so schnell wie nach einer Aktivierung durch die unter den Punkten 1. bis 5. beschriebenen Methoden.

Dabei findet die Aktivierung des Erdalkalimetalls insbesondere in nicht-koordinierenden Lösemitteln statt. Das aktivierte Erdalkalimetall soll keine störenden Verunreinigungen, beispielsweise lod, enthalten.

Dabei wird erfindungsgemäß metallisches Erdalkalimetall, bevorzugt metallisches Magnesium, in Gegenwart eines oder mehrerer nicht-koordinierender Lösemittel mit Aluminiumverbindungen der allgemeinen Formel AlR₃₋ₙHalₙ, nachfolgend Aktivierungsmittel genannt, in Kontakt gebracht. Dadurch wird das metallische Erdalkalimetall aktiviert, das heißt, seine Reaktionsfähigkeit wird gesteigert.

Dabei sind hier wie auch nachfolgend zu verstehen:
der Rest R als Alkyl- oder Aryl- Rest, bevorzugt als Alkyl-Rest, besonders bevorzugt als Alkyl-Rest mit 1 bis 8 C-Atomen;
der Rest Hal als Halogen-Rest, vorzugsweise als Chlor-, Brom- oder lod-Rest;
n als Zahl, wobei 0 ≤ n ≤ 2 ist.

Es wurde überraschenderweise gefunden, dass die erfindungsgemäßen Aktivierungsmittel bereits in sehr niedrigen Konzentrationen, beispielsweise in Konzentrationen von 0,001 bis 2 mol%, bevorzugt in Konzentrationen von 0,1 bis 1,5 mol%, bezogen auf eingesetztes Erdalkalimetall, in der Lage sind, die Reaktionsfähigkeit des Erdalkalimetalls derartig zu steigern, dass die gewünschten Produkte, insbesondere Dialkylmagnesiumverbindungen und Magnesiumalkoholate, ohne Induktionsperiode und in hohen Ausbeuten synthetisiert werden können. Es können prinzipiell aber auch höhere Mengen an Aktivierungsmittel, beispielsweise 10 oder sogar über 20 mol% eingesetzt werden. Die erhöhte Zugabe von Aktivierungsmittel hat den Vorteil, dass die Viskosität der Alkoxidlösungen erniedrigt wird. Dies macht diese Lösungen leichter förderbar.

Das Aktivierungsmittel wird vorzugsweise zur Suspension des Erdalkalimetalls im nicht-koordinierenden Lösemittel gegeben.

Das erfindungsgemäße Verfahren zur Herstellung des aktivierten Erdalkalimetalls wird allgemein wie nachfolgend beschrieben durchgeführt, ohne die Erfindung darauf einzuschränken:
Erdalkalimetall wird in einem nicht-koordinierenden Lösemittel, bevorzugt in aromatischen oder aliphatischen Kohlenwasserstoffen, besonders bevorzugt in Toluol, n-Hexan, n-Octan oder Mischungen von mindestens zwei von diesen, vorgelegt und unter Rühren mit dem Aktivierungsmittel versetzt. Dabei ist es wichtig, dass diese Operationen unter Ausschluss von Luft und Feuchtigkeit, bevorzugt unter Inertgas, besonders bevorzugt unter Argon, Helium oder Stickstoff, vorgenommen werden. Auf diese Art und Weise wird vermieden, dass sich die in der Regel luftempfindlichen Aktivierungsmittel teilweise oder ganz zersetzen. Unter Inertbedingungen werden daher deutlich geringere Mengen an Aktivierungsmittel benötigt, als dies in Anwesenheit von Sauerstoff, Wasser oder anderen reaktiven Stoffen der Fall ist.

Der Metallaktivierungsprozess erfolgt bei Temperaturen von 0 bis 150°C, bevorzugt von 20 bis 110°C. Die Kontaktzeit beträgt von einer Minute bis 5 Stunden, bevorzugt von 5 Minuten bis einer Stunde. Die Kontaktzeit ist dabei abhängig von der Konzentration des Aktivierungsmittels und der Beschaffenheit, das heißt der Dicke und Kompaktheit, der das Metall passivierenden Schicht.

Es wird das erfindungsgemäße aktivierte Erdalkalimetall erhalten.

Als Erdalkalimetall wird bevorzugt handelsübliches Magnesium, vorzugsweise gespant, granuliert oder in Pulverform, eingesetzt.

Als Aktivierungsmittel werden die schon beschriebenen Verbindungen der Formel AIR₃₋ₙHalₙ verwendet. Bevorzugt ist das Aktivierungsmittel ausgewählt aus Trimethylaluminium (TMA), Triethylaluminium (TEA), Tributylaluminium (TBA), Ethylaluminiumdichlorid (EADC) und/oder Diethylaluminiumchlorid (DEAC) oder Mischungen von zwei oder mehreren dieser Verbindungen. Diese Verbindungen sind in kommerziellen Mengen als Reinstoffe oder als Lösungen in Kohlenwasserstoffen erhältlich.

Das erfindungsgemäße aktivierte Erdalkalimetall kann beispielsweise wie folgt eingesetzt werden, ohne die Erfindung darauf einzuschränken:
Nach erfolgter Aktivierung kann mit der Dosierung des Alkohols oder des Gemisches von zwei oder mehreren Alkoholen oder des Alkylhalogenids oder des Gemisches von zwei oder mehreren Alkylhalogeniden begonnen werden. Die Dosierung erfolgt je nach Ansatzgröße und sonstigen apparativen Begleitumständen über einen Zeitraum von wenigen Minuten bis einigen Stunden. Typischerweise sind ein bis zwei Stunden sinnvoll. Die Reaktion erfolgt im Temperaturbereich zwischen Raumtemperatur (RT) und etwa 150°C. Wird sie bei Normaldruck gefahren, ist die Temperaturobergrenze durch den Siedepunkt des Lösemittels, also beispielsweise 110°C für Toluol oder ca. 65°C für n-Hexan gegeben.

Bevorzugt wird die Reaktion am Siedepunkt gefahren, wenn die thermische Stabilität des gebildeten Produkts dies zulässt.

Die Produktaufarbeitung erfolgt in Abhängigkeit von den chemisch-physikalischen Eigenschaften des Endproduktes. Unlösliche Produkte wie beispielsweise Magnesiumethoxid werden durch Fest-Flüssig-Trennung oder Totaleindampfung in reiner, das heißt lösemittelfreier Form gewonnen. Ist ein metallfreies Produkt erwünscht, muss auf vollständige Umsetzung geachtet werden. Der verwendete Alkohol, in Falle der Synthese von Magnesiumethoxid Ethanol, oder das organische Halogenid werden in mindestens der stöchiometrischen Menge, bevorzugt aber mit 0,1 bis 60 % Überschuss eingesetzt.

Falls eine im nicht-koordinierenden Lösemittel oder Lösemittelgemisch lösliche Verbindung, beispielsweise Magnesiumethylhexoxid hergestellt wird, kann das Reaktionsprodukt auf einfache Weise, beispielsweise durch Filtration, von unverändertem Magnesiummetall abgetrennt werden. In einem solchen Fall kann das Metall im Überschuss eingesetzt werden. Dies ist insbesondere dann sinnvoll, wenn die Produktlösung möglichst geringe Mengen an freiem Alkohol oder organischem Halogenid enthalten soll.

Gegenstand der Erfindung ist im Einzelnen:
- ein aktiviertes Erdalkalimetall, das mit organischen Halogeniden und/oder Alkoholen unverzögert reagiert;
- ein aktiviertes Erdalkalimetall, das durch Zugabe einer aktivierenden Verbindung in einem Lösemittel zum Erdalkalimetall erhältlich ist, wobei die aktivierende Verbindung der allgemeinen Formel AlR₃₋ₙHalₙ entspricht und das Lösemittel aus nicht-koordinierenden Lösemitteln ausgewählt ist.
- ein aktiviertes Erdalkalimetall, das durch Zugabe einer aktivierenden Verbindung der allgemeinen Formel AlR₃₋ₙHalₙ in einem nicht-koordinierenden Lösemittel zum Erdalkalimetall erhältlich ist, wobei die Verbindungen der allgemeinen Formel AlR_{3- n}Halₙ ausgewählt sind aus Trimethylaluminium (TMA), Triethylaluminium (TEA), Tributylaluminium (TBA), Ethylaluminiumdichlorid (EADC) und/oder Diethylaluminiumchlorid (DEAC) oder Mischungen aus zwei oder mehreren dieser Verbindungen;
- ein aktiviertes Erdalkalimetall, das durch Zugabe einer aktivierenden Verbindung der allgemeinen Formel AlR₃₋ₙHalₙ in einem nicht-koordinierenden Lösemittel zum Erdalkalimetall erhältlich ist, wobei die nicht-koordinierenden Lösemittel ausgewählt sind aus aromatischen oder aliphatischen Kohlenwasserstoffen oder aus Mischungen solcher Lösemittel;
- ein aktiviertes Erdalkalimetall, das durch Zugabe einer aktivierenden Verbindung der allgemeinen Formel AlR₃₋ₙHalₙ in einem nicht-koordinierenden Lösemittel zum Erdalkalimetall erhältlich ist, wobei die nicht-koordinierenden Lösemittel ausgewählt sind aus Toluol, n-Hexan, n-Octan oder Mischungen aus mindestens zwei dieser Lösemittel;
- ein aktiviertes Erdalkalimetall, das durch Zugabe einer aktivierenden Verbindung der allgemeinen Formel AlR₃₋ₙHalₙ in einem nicht-koordinierenden Lösemittel zum Erdalkalimetall erhältlich ist, wobei es sich bei dem Erdalkalimetall um Beryllium, Magnesium, Calcium, Strontium oder Barium, bevorzugt um Magnesium, Calcium oder Barium, besonders bevorzugt um Magnesium, handelt;
- eine Mischung, die ein erfindungsgemäßes aktiviertes Erdalkalimetall in einem Lösemittel, vorzugsweise einem nicht-koordinierenden Lösemittel, enthält;
- eine Mischung, die ein erfindungsgemäßes aktiviertes Erdalkalimetall in einem Lösemittel, vorzugsweise einem nicht-koordinierenden Lösemittel, und eine aktivierende Verbindung der allgemeinen Formel AlR₃₋ₙHalₙ enthält;
- ein Verfahren zur Herstellung von aktiviertem Erdalkalimetall durch Umsetzung von Erdalkalimetall mit Verbindungen der allgemeinen Formel AlR₃₋ₙHalₙ in nicht-koordinierenden Lösemitteln;
- ein Verfahren zur Herstellung von aktiviertem Erdalkalimetall durch Umsetzung von Erdalkalimetall mit Verbindungen der allgemeinen Formel AlR₃₋ₙHalₙ in nicht-koordinierenden Lösemitteln, das folgende Verfahrensschritte enthält:
   - Vorlage des Erdalkalimetalls in einem nicht-koordinierenden Lösemittel unter Inertbedingungen;
   - Zugabe einer oder mehrerer Verbindungen der allgemeinen Formel AlR₃₋ₙHalₙ unter Rühren;
- ein Verfahren zur Herstellung von aktiviertem Erdalkalimetall, wobei die Verbindungen der allgemeinen Formel AlR₃₋ₙHalₙ ausgewählt sind aus Trimethylaluminium (TMA), Triethylaluminium (TEA), Tributylaluminium (TBA), Ethylaluminiumdichlorid (EADC) und/oder Diethylaluminiumchlorid (DEAC) oder Mischungen aus zwei oder mehreren dieser Verbindungen;
- ein Verfahren zur Herstellung von aktiviertem Erdalkalimetall, wobei die nicht-koordinierenden Lösemittel ausgewählt sind aus aromatischen oder aliphatischen Kohlenwasserstoffe oder Mischungen solcher Lösemittel;
- ein Verfahren zur Herstellung von aktiviertem Erdalkalimetall, wobei die nicht-koordinierenden Lösemittel ausgewählt sind aus Toluol, n-Hexan, n-Octan oder Mischungen aus mindestens zwei dieser Lösemittel;
- ein Verfahren zur Herstellung von aktiviertem Erdalkalimetall, wobei es sich bei dem Erdalkalimetall um Beryllium, Magnesium, Calcium, Strontium oder Barium, bevorzugt um Magnesium, Calcium oder Barium, besonders bevorzugt um Magnesium, handelt;
- ein Verfahren zur Herstellung von aktiviertem Erdalkalimetall, wobei die Verbindungen der allgemeinen Formel AlR₃₋ₙHalₙ dem Erdalkalimetall in Konzentrationen von 0,001 bis 2 mol%, bevorzugt in Konzentrationen von 0,1 bis 1,5 mol%, bezogen auf eingesetztes Erdalkalimetall, zugegeben werden;
- ein Verfahren zur Herstellung von aktiviertem Erdalkalimetall, wobei die Verbindungen der allgemeinen Formel AlR₃₋ₙHalₙ dem Erdalkalimetall in Konzentrationen von bis zu 10 mol%, bevorzugt in Konzentrationen von bis zu 20 mol% und darüber zugegeben werden;
- ein Verfahren zur Herstellung von aktiviertem Erdalkalimetall, wobei der Metallaktivierungsprozess bei Temperaturen zwischen 0 und 150°C, bevorzugt zwischen 20 und 110°C erfolgt;
- ein Verfahren zur Herstellung von aktiviertem Erdalkalimetall, wobei die Kontaktzeit zwischen einer Minute und 5 Stunden, bevorzugt zwischen 5 Minuten und einer Stunde, beträgt;
- die Verwendung von aktiviertem Erdalkalimetall zur Herstellung von Erdalkalialkoxiden;
- die Verwendung von aktiviertem Erdalkalimetall zur Herstellung von Magnesium-, Calcium- und Bariumalkoxiden;
- die Verwendung von aktiviertem Magnesiummetall zur Herstellung von Dialkylmagnesiumverbindungen, Alkylmagnesiumhalogeniden, insbesondere Grignard-Verbindungen, Magnesiumalkoxiden, Magnesiumbis(dialkyl)amiden und Magnesiumhydrid.

Die Erfindung wird nachfolgend durch Beispiele erläutert, ohne sie darauf einzuschränken:

### Beispiel 1: Herstellung eines Magnesiumethoxid-Granulates durch Umsetzung von Magnesiumfeinspänen mit Ethanol in Toluolsuspension

In einem inertisierten 3-L-Doppelmantelreaktor, versehen mit Propellerrührer, Tropftrichter und Rückflusskühler werden 77,3 g (3180 mmol) Magnesiumfeinspäne (NF2 von Firma Minmet) in 1400 g Toluol vorgelegt. Dann wird unter Rühren auf 50°C erhitzt und 10 ml einer 25 %igen Triethylaluminiumlösung in Toluol zugegeben. Bei der angegebenen Temperatur wird 30 Minuten gerührt und dann mit der Zudosierung von wasserfreiem Ethanol begonnen. Es wurden insgesamt 307 g Ethanol (6660 mmol, 4,7 % Überschuss) über einen Zeitraum von 1,5 Stunden zugegeben. Die Umsetzung springt fast unverzögert an, was an Temperaturerhöhung und Gasentwicklung zu erkennen ist. Durch Gegenkühlung wird die Innentemperatur auf 65°C begrenzt. Bei Zugabeende haben sich 55 L Gas (≙ 72 % d.Th.) entwickelt.

Die Manteltemperatur wird dann auf 90°C erhöht. Nach einer Nachreaktionszeit von 100 Minuten kommt die Gasbildung zum Stillstand. Insgesamt haben sich 75,8 L Gas (≙ 99 % d.Th.) entwickelt.

Die Reaktionsmischung wird auf eine Glasfritte abgelassen und die Toluolphase abgetrennt. Der Filterrückstand wird mit Hexan gewaschen und zunächst bei Raumtemperatur vorgetrocknet. Zur Endtrocknung wird der Feststoff in einen 1 L-Rundkolben gefüllt und im Vakuum bei 110°C getrocknet.

Es werden 338 g (93% d.Th.) eines granulierten Materials mit einem Staubanteil von 1,5% erhalten.

Die Reaktionsbedingungen und -ergebnisse sind in der Tabelle 1 und der Tabelle 2 festgehalten.

### Beispiel 2: Herstellung eines Magnesiumethoxid-Granulates durch Umsetzung von Magnesiumgranulat mit Ethanol in Toluolsuspension

In derselben Apparatur wie in Beispiel 1 wird Magnesiumgranulat mit Triethylaluminium in Toluol aktiviert und mit Ethanol umgesetzt. Die Reaktionsbedingungen und -ergebnisse sind in der Tabelle 1 und der Tabelle 2 festgehalten.

### Beispiel 3: Herstellung eines Magnesiumethoxid-Granulates durch Umsetzung von Magnesiumgrobspänen mit Ethanol in Toluolsuspension:

In derselben Apparatur wie in Beispiel 1 werden Magnesiumgrobspäne mit Triethylaluminium in Toluol aktiviert und mit Ethanol umgesetzt. Die Reaktionsbedingungen und -ergebnisse sind in der Tabelle 1 und der Tabelle 2 festgehalten.

### Beispiel 4: Herstellung von Magnesiumethylhexoxid in Toluol:

In einem 1-L-Doppelmantelreaktor werden 11,2 g Magnesiumspäne in 220 ml Toluol vorgelegt. Nach Aufheizen auf 50°C werden 5 ml einer 25%igen Triethylaluminiumlösung in Toluol zugegeben und 30 Minuten gerührt. Dann wird auf 110°C aufgeheizt und 55,5 g 2-Ethylhexanol innerhalb von 2 Stunden zugetropft. Die Reaktion springt sofort an. Nach insgesamt 3 Stunden haben sich 4,9 L Gas gebildet.

Nach Abfühlen auf 80°C wird über eine G3-Fritte filtriert. Die Ausbeute beträgt 269 g einer 19%igen viskosen Lösung von Magnesiumethylhexoxid in Toluol.

### Beispiel 5: Herstellung von Bariumethylhexoxid in n-Octan:

In einem 250-ml-Glaskolben werden 66 mmol Bariumspäne in 150 ml Octan vorgelegt und mit 0,5 ml einer 25%igen Triethylaluminiumlösung in Toluol versetzt. Es wird auf 130°C erwärmt und 122 mmol Ethylhexanol zugegeben. Nach einer Reaktionszeit von 5 Stunden bei 130°C haben sich 1,6 L Gas entwickelt.

Nach Filtration werden 162 g einer 13,5%igen Lösung von Bariumethylhexoxid in n-Octan gewonnen.

### Beispiel 6: Herstellung von Calciumethylhexoxid in n-Octan

In derselben Apparatur wie Beispiel 5 werden 65 mmol Calciumspäne in 120 g Octan vorgelegt, mit 1,5 mol% Triethylaluminiumlösung aktiviert und bei 120 bis 130°C mit 140 mmol Ethylhexanol versetzt. Nach 6 Stunden Reaktionszeit entwickelt sich kein Gas mehr.

Es werden 145 g einer 4%igen Lösung von Calciumethylhexoxid in n-Octan gewonnen.

### Vergleichsbeispiell: Herstellung von Magnesiumethoxid durch Umsetzung von Magnesium in wasserfreien Ethanol:

In der Apparatur aus Beispiel 1 werden 76 g Magnesiumfeinspäne in 2,05 kg wasserfreiem Ethanol (Wassergehalt nach Karl Fischer: 36 ppm) suspendiert und die Manteltemperatur innerhalb von 20 Minuten auf 90°C angehoben, so dass der Reaktorinhalt siedet. Nach insgesamt 70 Minuten haben sich knapp 20 L Gas (ca. 26 % d.Th.) entwickelt. Danach nimmt die Gasbildungsrate stark ab. Innerhalb weiterer 4 Stunden Rückflusskochen werden nur noch 21 L Gas freigesetzt. Daraufhin werden 0,8 g lod und nach einer weiteren Stunde 1,8 g Eisenchlorid zugegeben. Die Gasbildungsrate nahm daraufhin nur kurzzeitig zu. Nach insgesamt 15- bzw. 20-stündigem Refluxieren haben sich 57,6 bzw. 65,5 L Gas gebildet (ca. 77 bzw. 87 % d.Th.).

Die grau-weiße Suspension wird filtriert und bei 110°C vakuumgetrocknet. Das fertige Produkt besteht überwiegend aus einem feinen Pulver mit sehr hohem Staubanteil. Neben Klumpen enthält es noch etwa 10 % nicht umgesetztes metallisches Magnesium.

Die wichtigsten Reaktionsbedingungen und -ergebnisse sind in der Tabelle 1 und der Tabelle 2 festgehalten.

**Tabelle 1: Herstellung von Magnesiumethoxid-Granulat**

| Beispiel | Magnesium | | Toluol | Ethanol | | TEA* | |
|---|---|---|---|---|---|---|---|
| | Art | Menge (g) | (g) | (g) | (% d.Th.) | (g) | (mol %) |
| 1 | Feinspäne | 77,3 | 1400 | 307 | 105 | 2,5 | 0,7 |
| 2 | Granulat | 80,5 | 1550 | 355 | 116 | 1,1 | 0,3 |
| 3 | Grobspäne | 65,3 | 1630 | 267 | 108 | 2,3* | 1,2* |
| Vgl.1 | Feinspäne | 76,0 | 0 | 2050 | 712 | - | - |

| | | | | | | | |
|---|---|---|---|---|---|---|---|
| * Trimethylaluminium | | | | | | | |

**Tabelle 2: Herstellergebnisse**

| Beispiel | Reaktionszeit | Reaktionstemp. | Gasmenge (I) | Produktmenge (g) | Aussehen | Staubanteil (%) |
|---|---|---|---|---|---|---|
| | (h) | (°C) | L | (g) | | (%) |
| 1 | 3,2 | 50 - 90 | 75,8 | 338 | Granulat | 1,5 |
| 2 | 8 | 60 -105 | 80,1 | 356 | Granulat | <1 |
| 3 | 4 | 90 -108 | 64,3 | 281 | Granulat | 3 |
| Vgl. 1 | 20 | 50 - 78 | 65,5 | 305 | Pulver mit Klumpen | 72 |

Gespantes Magnesium benötigt nach dem erfindungsgemäßen Verfahren etwa 3 bis 4 Stunden für die vollständige Umsetzung zu Magnesiumethoxid. Wird kompakteres, granuliertes Magnesiummetall eingesetzt, sind die Reaktionszeiten länger, wie aus Beispiel 2 hervorgeht. Der Staubanteil, das heißt der Anteil an Endprodukt, dessen Korngröße < 0,2 mm ist, liegt unter 1 %.

## Patentansprüche

1. Verfahren zur Herstellung von aktiviertem Erdalkalimetall, **dadurch gekennzeichnet, dass** das Erdalkalimetall in Gegenwart eines oder mehrerer nicht-koordinierender Lösemittel in Form von aromatischen oder aliphatischen Kohlenwasserstoffen oder Mischungen dieser Lösemittel mit Aluminiumverbindungen der allgemeinen Formel AlR₃₋ₙHalₙ in Kontakt gebracht wird, wobei n eine Zahl von 0 ≤ n ≤ 2 ist und das Erdalkalimetall mit organischen Halogeniden und/oder Alkoholen unverzögert reagiert.

2. Verfahren nach Anspruch 1, **dadurch gekennzeichnet, dass** es folgende Verfahrensschritte enthält:
- Vorlage des Erdalkalimetalls in einem nicht-koordinierenden Lösemittel unter Inertbedingungen;
- Zugabe einer oder mehrerer Verbindungen der allgemeinen Formel AlR₃₋ₙHalₙ unter Rühren;

3. Verfahren nach Anspruch 1 oder 2, **dadurch gekennzeichnet, dass** die Verbindungen der allgemeinen Formel AlR₃₋ₙHalₙ ausgewählt sind aus Trimethylaluminium (TMA), Triethylaluminium (TEA), Tributylaluminium (TBA), Ethylaluminiumdichlorid (EADC) und/oder Diethylaluminiumchlorid (DEAC) oder Mischungen aus zwei oder mehreren dieser Verbindungen.

4. Verfahren nach mindestens einem der Ansprüche 1 bis 3, **dadurch gekennzeichnet, dass** die nicht-koordinierenden Lösemittel ausgewählt sind aus aromatischen oder aliphatischen Kohlenwasserstoffe oder Mischungen dieser Lösemittel.

5. Verfahren nach mindestens einem der Ansprüche 1 bis 4, **dadurch gekennzeichnet, dass** die nicht-koordinierenden Lösemittel ausgewählt sind aus Toluol, n-Hexan, n-Octan oder Mischungen aus mindestens zwei dieser Lösemittel.

6. Verfahren nach mindestens einem der Ansprüche 1 bis 5 **dadurch gekennzeichnet, dass** es sich bei dem Erdalkalimetall um Beryllium, Magnesium, Calcium, Strontium oder Barium, bevorzugt um Magnesium, Calcium oder Barium, besonders bevorzugt um Magnesium, handelt.

7. Verfahren nach mindestens einem der Ansprüche 1 bis 6 **dadurch gekennzeichnet, dass** die Verbindungen der allgemeinen Formel AlR₃₋ₙHalₙ dem Erdalkalimetall in Konzentrationen von 0,001 bis 2 mol%, bevorzugt in Konzentrationen von 0,1 bis 1,5 mol%, bezogen auf eingesetztes Erdalkalimetall, zugegeben werden.

8. Verfahren nach mindestens einem der Ansprüche 1 bis 7 **dadurch gekennzeichnet, dass** die Verbindungen der allgemeinen Formel AlR₃₋ₙHalₙ dem Erdalkalimetall in Konzentrationen von bis zu 10 mol%, bevorzugt in Konzentrationen von bis zu 20 mol% und darüber zugegeben werden.

9. Verfahren zur Herstellung nach mindestens einem der Ansprüche 1 bis 8, **dadurch gekennzeichnet, dass** der Metallaktivierungsprozess bei Temperaturen zwischen 0 und 150°C, bevorzugt zwischen 20 und 110°C erfolgt.

10. verfahren zur Herstellung nach mindestens einem der Ansprüche 1 bis 9, **dadurch gekennzeichnet, dass** die Kontaktzeit zwischen einer Minute und 5 Stunden, bevorzugt zwischen 5 Minuten und einer Stunde, beträgt.

11. Verwendung von aktiviertem Erdalkalimetall gemäß Anspruch 1, zur Herstellung von Erdalkalialkoxiden.

12. Verwendung nach Anspruch 11 zur Herstellung von Magnesium-, Calcium- und Bariumalkoxiden, bevorzugt zur Herstellung von Magnesiumalkoxiden.

13. Verwendung nach Anspruch 12 zur Herstellung von Dialkylmagnesiumverbindungen, Alkylmagnesiumhalogeniden, insbesondere Grignard-Verbindungen, Magnesiumalkoxiden, Magnesiumbis(dialkyl)amiden und Magnesiumhydrid.

## Claims

1. A process for the preparation of activated alkaline-earth metal, **characterised in that** the alkaline-earth metal in the presence of one or more non-coordinating solvents in the form of aromatic or aliphatic hydrocarbons or mixtures of these solvents is brought into contact with aluminium compounds of the general formula AlR₃₋ₙHalₙ, wherein n is a number from 0 ≤ n ≤ 2, and the alkaline-earth metal reacts with organic halides and/or alcohols instantaneously.

2. A process according to claim 1, **characterised in that** it contains the following process steps:
- presenting the alkaline-earth metal in a non-coordinating solvent under inert conditions;
- adding one or more compounds of the general formula AlR₃₋ₙHalₙ while stirring.

3. A process according to claim 1 or 2, **characterised in that** the compounds of the general formula AlR₃₋ₙHalₙ are selected from trimethylaluminium (TMA), triethylaluminium (TEA), tributylaluminium (TBA), ethyl-aluminium dichloride (EADC) and/or diethylaluminium chloride (DEAC) or mixtures of two or more of these compounds.

4. A process according to at least one of claims 1 to 3, **characterised in that** the non-coordinating solvents are selected from aromatic or aliphatic hydrocarbons or mixtures of these solvents.

5. A process according to at least one of claims 1 to 4, **characterised in that** the non-coordinating solvents are selected from toluene, n-hexane, n-octane or mixtures of at least two of these solvents.

6. A process according to at least one of claims 1 to 5, **characterised in that** the alkaline-earth metal is beryllium, magnesium, calcium, strontium or barium, preferably magnesium, calcium or barium, particularly preferably magnesium.

7. A process according to at least one of claims 1 to 6, **characterised in that** the compounds of the general formula AlR₃₋ₙHalₙ are added to the alkaline-earth metal in concentrations of 0.001 to 2 mole%, preferably in concentrations of 0.1 to 1.5 mole%, based on alkaline-earth metal used.

8. A process according to at least one of claims 1 to 7, **characterised in that** the compounds of the general formula AlR₃₋ₙHalₙ are added to the alkaline-earth metal in concentrations of up to 10 mole%, preferably in concentrations of up to 20 mole% and more.

9. A process for preparation according to at least one of claims 1 to 8, **characterised in that** the metal activation process takes place at temperatures of between 0 and 150°C, preferably between 20 and 110°C.

10. A process for preparation according to at least one of claims 1 to 9, **characterised in that** the contact period is between one minute and 5 hours, preferably between 5 minutes and one hour.

11. Use of activated alkaline-earth metal according to claim 1 for the preparation of alkaline-earth alkoxides.

12. Use according to claim 11 for the preparation of magnesium, calcium and barium alkoxides, preferably for the preparation of magnesium alkoxides.

13. Use according to claim 12 for the preparation of dialkylmagnesium compounds, alkylmagnesium halides, in particular Grignard compounds, magnesium alkoxides, magnesium bis(dialkyl)amides and magnesium hydride.

## Revendications

1. Procédé de préparation d'un métal alcalino-terreux activé, **caractérisé en ce que**, en présence d'un ou de plusieurs solvants non-coordinants, sous forme d'hydrocarbures aromatiques ou aliphatiques ou de mélanges de tels solvants, on met le métal alcalino-terreux en contact avec un composé de l'aluminium de formule générale AlR₃₋ₙHalₙ dans laquelle l'indice n est un nombre tel que 0 ≤ n ≤ 2, et l'on fait sans tarder réagir le métal alcalino-terreux avec un halogénure organique et/ou un alcool.

2. Procédé conforme à la revendication 1, **caractérisé en ce qu'**il comporte les étapes suivantes :
- mettre le métal alcalino-terreux dans un solvant non-coordinant, dans des conditions inertes ;
- et ajouter, tout en agitant le tout, un ou plusieurs composés de formule générale AlR₃₋ₙHalₙ.

3. Procédé conforme à la revendication 1 ou 2, **caractérisé en ce que** les composés de formule générale AlR₃₋ₙHalₙ sont choisis parmi les triméthyl-aluminium (TMA), triéthyl-aluminium (TEA), tributyl-aluminium (TBA), dichlorure d'éthyl-aluminium (EADC) et/ou chlorure de diéthyl-aluminium (DEAC), et les mélanges de deux de ces composés ou plus.

4. Procédé conforme à l'une au moins des revendications 1 à 3, **caractérisé en ce que** les solvants non-coordinants sont choisis parmi les hydrocarbures aromatiques ou aliphatiques et les mélanges de tels solvants.

5. Procédé conforme à l'une au moins des revendications 1 à 4, **caractérisé en ce que** les solvants non-coordinants sont choisis parmi le toluène, le n-hexane, le n-octane, et les mélanges d'au moins deux de ces solvants.

6. Procédé conforme à l'une au moins des revendications 1 à 5, **caractérisé en ce que** le métal alcalino-terreux est du béryllium, du magnésium, du calcium, du strontium ou du baryum, de préférence du magnésium, du calcium ou du baryum, et en particulier du magnésium.

7. Procédé conforme à l'une au moins des revendications 1 à 6, **caractérisé en ce que** les composés de formule générale AlR₃₋ₙHalₙ sont ajoutés au métal alcalino-terreux en des concentrations de 0,001 à 2 % en moles, et de préférence de 0,1 à 1,5 % en moles, par rapport au métal alcalino-terreux utilisé.

8. Procédé conforme à l'une au moins des revendications 1 à 7, **caractérisé en ce que** les composés de formule générale AlR₃₋ₙHalₙ sont ajoutés au métal alcalino-terreux en des concentrations pouvant valoir jusqu'à 10 % en moles, et de préférence jusqu'à 20 % en moles et plus.

9. Procédé de préparation conforme à l'une au moins des revendications 1 à 8, **caractérisé en ce que** l'opération d'activation du métal est effectuée à des températures comprises entre 0 et 150 °C, et de préférence entre 20 et 110 °C.

10. Procédé de préparation conforme à l'une au moins des revendications 1 à 9, **caractérisé en ce que** le temps de contact vaut entre 1 minute et 5 heures, et de préférence entre 5 minutes et 1 heure.

11. Utilisation d'un métal alcalino-terreux activé conformément à la revendication 1 en vue de la préparation d'alcoolates de métal alcalino-terreux.

12. Utilisation conforme à la revendication 11, en vue de la préparation d'alcoolates de magnésium, de calcium ou de baryum, et de préférence en vue de la préparation d'alcoolates de magnésium.

13. Utilisation conforme à la revendication 12, en vue de la préparation de composés de type dialkyl-magnésium, d'halogénures d'alkyl-magnésium, en particulier de réactifs de Grignard, d'alcoolates de magnésium, de bis(dialkyl-amidures) de magnésium ou d'hydrure de magnésium.
